# EUROPÄISCHE PATENTSCHRIFT

(11) **EP 3 544 924 B1**
(45) Veröffentlichungstag und Bekanntmachung des Hinweises auf die Patenterteilung: **02.04.2025**
(21) Anmeldenummer: 17811867.5
(22) Anmeldetag: 22.11.2017
(51) Int. Cl.: B67C 3/22, A61L 2/20, B29C 49/46, F16L 39/06

(54) **VERTEILVORRICHTUNG ZUM VERTEILEN VON FLIESSFÄHIGEN MEDIEN**
DISTRIBUTOR DEVICE FOR DISTRIBUTING FLOWABLE MEDIA
DISPOSITIF DE RÉPARTITION POUR RÉPARTIR DES SUBSTANCES FLUIDES

(30) Priorität: 22.11.2016 DE 102016122542
(43) Veröffentlichungstag der Anmeldung: 02.10.2019
(73) Patentinhaber: Krones AG, 93073 Neutraubling (DE)
(72) Erfinder: SUPPES, Waldemar, 93073 Neutraubling (DE); GELTINGER, Florian, 93073 Neutraubling (DE); HÖLLRIEGL, Thomas, 93073 Neutraubling (DE); BRUNNER, Andreas, 93073 Neutraubling (DE); SÖLLNER, Jürgen, 93073 Neutraubling (DE)
(74) Vertreter: Hannke Bittner & Partner mbB Regensburg
(86) Internationale Anmeldenummer: PCT/EP2017/080047
(87) Internationale Veröffentlichungsnummer: WO 2018/095970

(56) Entgegenhaltungen:
- EP-A1- 2 604 295
- EP-A1- 2 722 304
- DE-A1- 102006 053 193
- DE-A1- 102007 041 685

## Beschreibung

Die vorliegende Erfindung bezieht sich auf eine Vorrichtung und ein Verfahren zum Verteilen von fließfähigen Medien. Derartige Vorrichtungen und Verfahren sind aus dem Stand der Technik seit langem bekannt. So sind beispielsweise Drehverteiler bekannt, welche Flüssigkeit aus einem Tank auf eine Vielzahl von Füllleitungen verteilen. Auch im Bereich der Sterilisationstechnik sind derartige Verteilereinrichtungen bekannt, welche ein Sterilisationsmittel und insbesondere ein flüssiges oder gasförmiges Sterilisationsmittel auf eine Vielzahl von Ausgängen wie etwa Düsen verteilen.

Derartige Verteilereinrichtungen weisen dabei üblicherweise Drehverteiler auf, da im Stand der Technik üblicherweise die zu behandelnden Behältnisse mittels eines drehbaren Trägers, das heißt entlang einer kreisförmigen Bahn, gefördert werden. So ist es beispielsweise bekannt, dass ein Sterilisationsmedium, wie beispielsweise H₂O₂ über ein stehendes Dach einer Behandlungskammer zugeführt wird und über eine mit Hybridlagern drehbar aufgehängte Verteilerkammer an die einzelnen Behandlungsstationen weitergeleitet wird. Diese Vorgehensweise hat sich zwar technisch bewährt, ist jedoch relativ aufwendig und teuer. Insbesondere kommen bei diesen Lösungen auch Hybridlager zum Einsatz, welche für sich genommen bereits sehr hohe Preise aufweisen.

Daneben weisen derartige Konstruktionen auch eine aufwendige Anschlusskonstruktion zur Einbettung der Lager auf. Daneben sind derartige Lager auch empfindlich bei Fehlmontage.

Entsprechende Abdichtungen müssen derzeit auch nicht vollständig dichten, da beispielsweise ein Sterilisationsmedium in jedem Fall in die jeweilige Behandlungskammer strömt.

Eine Vorrichtung gemäß dem Oberbegriff des Anspruchs 1 ist aus dem Patentdokument EP 2 604 295 A1 bekannt.

Der vorliegenden Erfindung liegt daher die Aufgabe zugrunde, eine Vorrichtung zur Verfügung zu stellen, welche auf den Einsatz der oben beschriebenen teuren Lager verzichtet, und welche auf diese Weise günstiger herzustellen ist. Diese Aufgabe wird erfindungsgemäß durch den Gegenstand der unabhängigen Patentansprüche erreicht. Vorteilhafte Ausführungsformen und Weiterbildungen sind Gegenstand der Unteransprüche.

Eine erfindungsgemäße Vorrichtung zum Verteilen fließfähiger Medien auf Behältnisse weist die Merkmale des Anspruchs 1 auf.

Es wird daher im Gegensatz zum Stand der Technik vorgeschlagen, dass die Verteilereinrichtung nicht über das oben erwähnte teure Lager gestützt wird, sondern drehfest bzw. mechanisch mit dem Träger verbunden wird und damit durch diesen gestützt wird. Bevorzugt ist die Verteilereinrichtung mechanisch fest mit dem Träger verbunden. Dieser Träger kann dabei als scheibenförmiger Träger ausgeführt sein. Bevorzugt wird dieser Träger mittels einer zentralen Welle angetrieben. Bei den Behältnissen handelt es sich insbesondere um Vorformlinge, insbesondere um Kunststoffvorformlinge. Daneben könnten jedoch auch andere Behältnisse behandelt werden wie etwa Flaschen und dergleichen. Allgemein kann die Erfindung jedoch auch auf andere zu behandelnde Objekte bzw. Stückgüter Anwendung finden. Bei einer weiteren vorteilhaften Ausführungsform ist die oben erwähnte Transporteinrichtung Bestandteil einer Behandlungseinrichtung, insbesondere einer Einrichtung zum Sterilisieren von Preforms. Bei dem fließfähigen Medium handelt es sich insbesondere um (bevorzugt gasförmiges) H2O2.

Bei einer weiteren bevorzugten Ausführungsform ist zumindest die Vorrichtung in ihrer Gesamtheit in einem Reinraum angeordnet. Neben der genannten Vorrichtung können auch weitere Einrichtungen wie insbesondere aber nicht ausschließlich Transportsterne innerhalb des Reinraums angeordnet sein. Dieser Reinraum ist bevorzugt mit wenigstens einer Wandung gegenüber einer (unsterilen) Umgebung abgegrenzt.

Bevorzugt handelt es sich bei dem Träger um ein Transportrad, an dem besonders bevorzugt Halteelemente zum Halten von Behältnissen angeordnet sind. Bei diesen Halteelementen kann es sich beispielsweise um Greifklammern handeln, welche die Behältnisse greifen können, beispielsweise Greifklammern, welche die Behältnisse in einem Bereich von deren Mündungen greifen.

Bei einer vorteilhaften Ausführungsform ist das fließfähige Medium gasförmig oder flüssig. Besonders bevorzugt handelt es sich bei dem fließfähigen Medium um Wasserstoffperoxid (H₂O₂) oder Peressigsäure. Es wäre jedoch auch möglich, dass es sich bei dem fließfähigen Medium um eine Flüssigkeit handelt, beispielsweise um eine in die Behältnisse abzufüllende Flüssigkeit.

Bei einer weiteren vorteilhaften Ausführungsform ist die Verteilereinrichtung mittels mechanischer Befestigungselemente an dem Träger angeordnet. Bei diesen Befestigungselementen kann es sich beispielsweise um Befestigungsstangen handeln, welche die Verteilereinrichtung stützen.

Bei einer weiteren vorteilhaften Ausführungsform bildet das Gehäuse einen Reinraum aus, innerhalb dessen sterile Bedingungen (gegenüber einer Umgebung) herstellbar sind. Dabei kann dieser Reinraum bezüglich einander bewegliche Wandungen aufweisen, welche besonders bevorzugt bezüglich einander abgedichtet sind. Bei einer bevorzugten Ausführungsform bildet der oben erwähnte drehbare Träger auch eine den Reinraum begrenzende Wandung aus.

Bei einer weiteren vorteilhaften Ausführungsform ist die Zuführleitung wenigstens abschnittsweise drehfest angeordnet und die Verteilereinrichtung ist gegenüber der Zuführleitung drehbar. Bevorzugt ist die Verteilereinrichtung bezüglich einer stationären Drehachse drehbar. Bevorzugt handelt es sich bei dieser Drehachse um eine vertikal verlaufende Drehachse. Bevorzugt ist die Drehachse, um welche die Verteilereinrichtung drehbar ist, parallel zu der Drehachse, um welche der Träger drehbar ist und besonders bevorzugt fallen diese beiden Drehachsen zusammen.

Es wird daher vorgeschlagen, dass die Verteilerkammer bzw. Verteilereinrichtung fest mit dem rotierenden Behandlungsrad verbunden wird. Auf diese Weise kann auf eine Lagerung verzichtet werden. Eine Verbindung zwischen einem stehenden Dach und einer drehenden Verteilerkammer erfolgt, wie unten genauer beschrieben, bevorzugt über eine Dichteinrichtung beispielsweise einem Balg mit einem Wellendichtring.

Bei einer weiteren vorteilhaften Ausführungsform erstreckt sich die Zuführeinrichtung durch eine den Reinraum begrenzende Wandung. Besonders bevorzugt befindet sich die Dichteinrichtung wenigstens teilweise und bevorzugt vollständig innerhalb des besagten Reinraums. Bevorzugt weist die Vorrichtung Druckbeaufschlagungsmittel auf, um in dem Reinraum einen Überdruck gegenüber einem Umgebungsdruck zu erzeugen.

Bei einer weiteren vorteilhaften Ausführungsform dient die Abdichteinrichtung auch dazu, um ein Austreten des zu transportierenden Mediums in den Reinraum zu verhindern.

Bei einer weiteren vorteilhaften Ausführungsform weist der Eingang eine drehfest an der Verteilereinrichtung angeordnete Eingangsleitung auf, welche in Strömungsverbindung mit der Zuführleitung bringbar ist. Dabei ist es besonders bevorzugt denkbar, dass diese Eingangsleitung und die Zuführleitung den gleichen Strömungsquerschnitt aufweisen. Falls als Abdichteinrichtung ein Balg mit einem Wellendichtring verwendet wird, ist es denkbar, dass der Balg einen Toleranzausgleich übernimmt und der Wellendichtring die Abdichtungsfunktion. Vorteilhaft kann dabei mittels einer Drehmomentstütze ein Mitdrehen des Wellendichtrings vermieden werden.

Bei einer weiteren vorteilhaften Ausführungsform weist die Verteilereinrichtung einen Sammelraum bzw. eine Sammelkammer auf, welche zum Sammeln des von der Zuführeinrichtung zugeführten fließfähigen Mediums dient. Diese Sammelkammer kann dabei bevorzugt rotationssymmetrisch bezüglich einer vorgegebenen Achse und insbesondere einer Drehachse sein, um welche sich diese Sammelkammer im Betrieb dreht. Bevorzugt ist diese Drehachse parallel zu einer Drehachse des Trägers und fällt besonders bevorzugt mit dieser zusammen.

Weiterhin weist bevorzugt diese Sammelkammer eine Vielzahl von Öffnungen auf, an welche bevorzugt Leitungen angeschlossen werden können, welche zum Weitertransport des fließfähigen Mediums zu den einzelnen Behältnissen dienen.

Es wäre jedoch auch denkbar, dass einzelne dieser Leitungen das fließfähige Medium nicht zu den Behältnissen führen, sondern zu Bereichen der Vorrichtung. Auf diese Weise kann das fließfähige Medium auch zum Reinigen der Vorrichtung selbst genutzt werden, etwa zur Durchführung einer CIP (cleaning in place) - Reinigung. Dies ist insbesondere in Verbindung mit einem Reinraum sehr interessant, da es unter Umständen möglich ist, den Reinraum zu reinigen, ohne hierzu diesen Reinraum öffnen zu müssen.

Bei einer weiteren vorteilhaften Ausführungsform weist daher die Vorrichtung eine Reinigungseinrichtung zum Reinigen von Bestandteilen der Vorrichtung selbst auf. Diese Reinigungseinrichtung ist dabei bevorzugt derart ausgeführt, dass wenigstens ein Teil eines Reinigungsmediums auch über die Verteilereinrichtung gefördert wird.

Bei einer bevorzugten Ausführungsform sind daher dieser stehende und der drehende Teil und insbesondere die stehende Zuführleitung und der drehende Eingang durch wenigstens ein flexibles Element miteinander verbunden. Diese Flexibilität ist dabei insbesondere in Richtung dieser Drehachse gegeben.

Dabei ist es möglich, dass ein Ende dieses flexiblen Dichtelements fest fixiert wird und das andere drehbar gelagert ist. Bevorzugt ist dieses besagte Ende fest an einem Bereich der Zuführleitung fixiert und das andere Ende ist drehbar gegenüber der Eingangsleitung gelagert. Bevorzugt ist diese Eingangsleitung als Eingangsrohr ausgeführt.

Bei einer weiteren vorteilhaften Ausführungsform ist zwischen der Eingangsleitung und der Zuführleitung die Abdichteinrichtung angeordnet. Bevorzugt weist wie oben erwähnt die Abdichteinrichtung ein entlang der Drehachse der Verteilereinrichtung elastisches Element und/oder bevorzugt einen Faltenbalg auf. Dabei kann eine Dichtung beispielsweise durch eine Dichtlippe oder einen Faltenbalg erfolgen.

Bei einer weiteren vorteilhaften Ausführungsform ist der Faltenbalg aus einem Kunststoff ausgeführt. Bei diesem Kunststoff kann es sich besonders bevorzugt um PTFE handeln. Bei einer weiteren vorteilhaften Ausführungsform ist das flexible Dichtelement im Bereich des Wellendichtrings aus einem anderen Werkstoff gefertigt wie der oben erwähnte Balg.

Bei einer weiteren vorteilhaften Ausführungsform ist der Bereich der Abdichtung aus einem Kunststoff hergestellt und besonders bevorzugt im Wesentlichen aus PTFE (Polytetraflourethylen). Besonders bevorzugt ist dieser Kunststoff durch ein Material verstärkt, welches aus einer Gruppe von Materialien ausgewählt ist, welche Peek (Polyetheretherketon), PI (Polyimide) und PAI (Polyamidimide) enthält.

Bei einer weiteren vorteilhaften Ausführungsform ist das flexible Dichtelement mehrteilig und besonders bevorzugt zweiteilig ausgeführt. Dabei kann ein Teil dieses Dichtelements beispielsweise an der Zuführleitung befestigt sein, während der zweite Teil gegenüber der Eingangsleitung gleitet und/oder drehbar ist.

Dabei ist es weiterhin möglich, dass der Bereich der Abdichtung über eine Abstützung in seiner Position gehalten wird.

Bei einer weiteren vorteilhaften Ausführungsform verbindet das elastische Element die Zuführleitung mit dem Eingang der Verteilereinrichtung und insbesondere mit einer Eingangsleitung des Eingangs.

Weiterhin ist es besonders bevorzugt denkbar, dass ein Teil des besagten Faltenbalgs an einem Element fest angeordnet ist und bevorzugt ein anderes Ende gegenüber einem weiteren Teil gleitet bzw. drehbar angeordnet ist.

Bei einer weiteren vorteilhaften Ausführungsform weist die Abdichteinrichtung eine Lagerungseinrichtung zum drehbaren Lagern wenigstens eines Elements des Eingangs oder der Zuführleitung auf. Insbesondere weist die Abdichteinrichtung eine Lagerungseinrichtung zum drehbaren Lagern der Eingangsleitung auf. Hierbei kann es sich insbesondere um eine Gleitlagerungseinrichtung handeln.

Auf diese Weise kann eine besonders abdichtfähige Abdichtung geschaffen werden.

Bei einer weiteren vorteilhaften Ausführungsform ist die Lagerungseinrichtung als Wellendichtring ausgebildet. Ein Wellendichtring hat sich besonders bewährt, um die drehbare Lagerung bei gleichzeitiger Aufrechterhaltung einer hohen Dichtwirkung zu erreichen.

Bei einer weiteren vorteilhaften Ausführungsform besteht der Wellendichtring aus einem anderen Material wie der erwähnte Faltenbalg. Dabei ist es insbesondere denkbar, dass das flexible Dichtelement wenigstens zweiteilig ausgeführt ist. Bevorzugt kann die Abdichteinrichtung eine umlaufende Dichtlippe aufweisen. Bevorzugt weist die Dichtlippe einen gekrümmten Abschnitt auf.

Bei einer weiteren vorteilhaften Ausführungsform sind die Zuführleitung und die Verteilereinrichtung berührungslos ausgebildet. Dies bedeutet, dass die Verbindung zwischen der stehenden Zuführleitung und der rotierenden Verteilerkammer berührungslos erfolgt, so dass die ortsfeste Zuführleitung die rotierende Verteilerkammer nicht berührt. Bevorzugt ist zwischen der stehenden und der drehenden Komponente ein Spalt von kleiner 15 mm, bevorzugt von kleiner 10 mm und besonders bevorzugt von kleiner 6 mm vorhanden.

Bei einer weiteren vorteilhaften Ausführungsform erfolgt eine Abdichtung zwischen der Zuführleitung und der Verteilereinrichtung durch eine berührungslose Labyrinth-Dichtung. Bevorzugt ist daher einer Abdichteinrichtung zwischen der Zuführleitung und der Verteilereinrichtung labyrinthartig ausgebildet. Besonders bevorzugt ist die Labyrinth-Dichtung dabei zweiteilig ausgebildet, wobei ein erster Teil der Labyrinth-Dichtung an der stehenden Zuführleitung angeordnet ist und ein zweiter Teil der Labyrinth-Dichtung an der drehenden Verteilereinrichtung. Bevorzugt sind die stehende und drehende Kontur des Labyrinths bzw. der Labyrinth-Dichtung im Wesentlichen komplementär ausgebildet. Bevorzugt kann durch die Labyrinth-Dichtung ein Leckagemassenstrom von kleiner 15% des Gesamtmassenstroms entweichen.

Die Labyrinth-Dichtung hat dabei insbesondere den Vorteil, dass hierdurch, auch im Vergleich zu der oben erwähnten Balglösung, nochmals Kosten eingespart werden können. Zudem ist durch die fehlende Berührung auch keine Reibung bzw. kein Abrieb mehr vorhanden, so dass auch der Verschleiß erheblich reduziert werden kann.

Die vorliegende Erfindung ist weiterhin auf ein Verfahren mit den Merkmalen des Anspruchs 14 gerichtet.

Es wird daher auch verfahrensseitig vorgeschlagen, dass die Verteilereinrichtung nicht wie im Stand der Technik bekannt, mittels Lagern, beispielsweise Wälzlagern, gelagert wird, sondern die Stützfunktion auch hier durch den Träger vorgenommen wird.

Bevorzugt werden die Behältnisse mit dem fließfähigen Medium beaufschlagt, beispielsweise mit einen Sterilisationsmittel beaufschlagt oder aber es wird ein fließfähiges Medium wie etwa ein Getränk in die Behältnisse eingeführt.

Bei einer weiteren vorteilhaften Ausführungsform wird das fließfähige Medium von der Verteilereinrichtung über eine Vielzahl von Leitungen zu den Behältnissen geführt, beispielsweise um diese zu sterilisieren.

Weitere Vorteile und Ausführungsformen ergeben sich aus den beigefügten Zeichnungen:
Darin zeigen:
- Fig. 1a bis 1c: drei Darstellungen einer Vorrichtung nach dem Stand der Technik;
- Fig. 2: eine Darstellung einer erfindungsgemäßen Vorrichtung;
- Fig. 3: eine weitere detailliertere Darstellung einer erfindungsgemäßen Vorrichtung;
- Fig. 4: eine Detaildarstellung der in Fig. 3 gezeigten Vorrichtung;
- Fig. 5: eine weitere Ausgestaltung einer erfindungsgemäßen Vorrichtung bei Verwendung eines Faltenbalgs;
- Fig. 6: eine Detaildarstellung der in Fig. 5 gezeigten Darstellung;
- Fig. 7: eine weitere Ausführungsform einer erfindungsgemäßen Vorrichtung bei Verwendung einer Labyrinth-Dichtung; und
- Fig. 8: eine Detaildarstellung der in Fig. 7 gezeigten Darstellung.

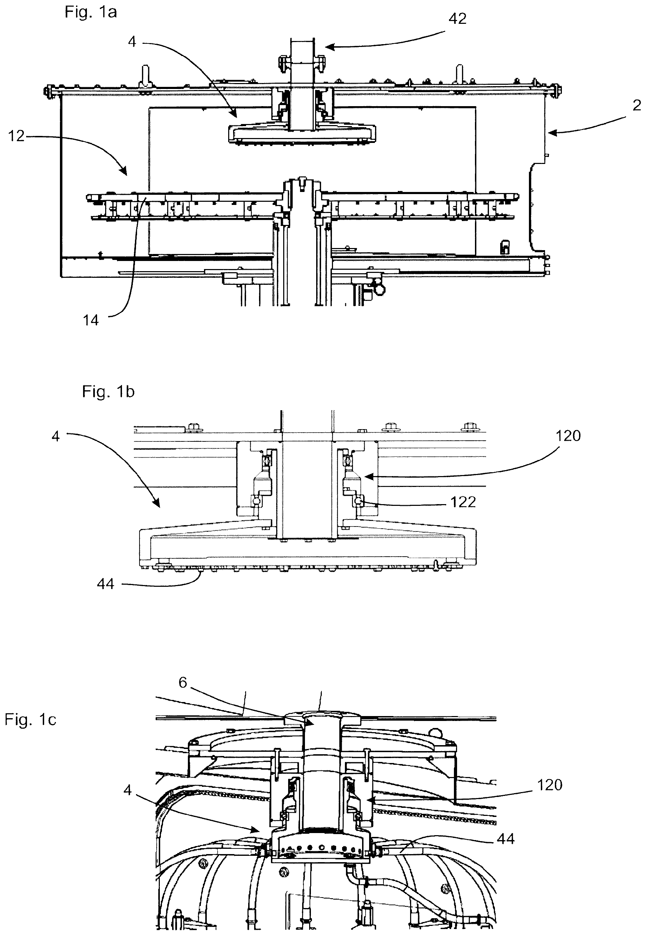

Die Fig. 1a zeigt eine Darstellung einer Vorrichtung nach dem Stand der Technik. Dabei ist eine Zuführleitung 42 vorgesehen, über welche einer Verteilerreinrichtung 4 ein fließfähiges Medium zugeführt wird. Das Bezugszeichen 12 bezieht sich auf eine Transporteinrichtung, welche einen drehbaren Träger aufweist, an dem auch die einzelnen Behältnisse gehalten werden können. Das Bezugszeichen 2 kennzeichnet ein Gehäuse, innerhalb dessen die Behältnisse sterilisiert werden können.

Fig. 1b zeigt eine Detaildarstellung der in Fig. 1a gezeigten Vorrichtung. Man erkennt hier, dass die Verteilereinrichtung 4 mittels eines Wälzlagers 120 mit Lagerkörpern 122 an der Decke der Vorrichtung gelagert ist. Diese Lagerung ist dabei relativ aufwendig.

Fig. 1c zeigt eine weitere Darstellung einer Vorrichtung nach dem internen Stand der Technik der Anmelderin. Auch hier ist die Lagerungseinrichtung 120 dargestellt, welche zum Lagern der Verteilereinrichtung 4 dient. Auch ist eine Zuführleitung 6 vorgesehen, welche auch hier stehend angeordnet ist.

Das Bezugszeichen 44 bezieht sich auf eine Leitungsverbindung, mittels derer das fließfähige Medium letztlich den Behältnissen zugeführt werden kann. Das Bezugszeichen 46 bezieht sich auf eine Eingangsleitung, welche einen Bestandteil des Eingangs 42 bildet.

Zum Dichten können bei der in Fig. 1b gezeigten Ausführungsform sowohl Spaltdichtungen als auch Hybridlager oder beides eingesetzt werden.

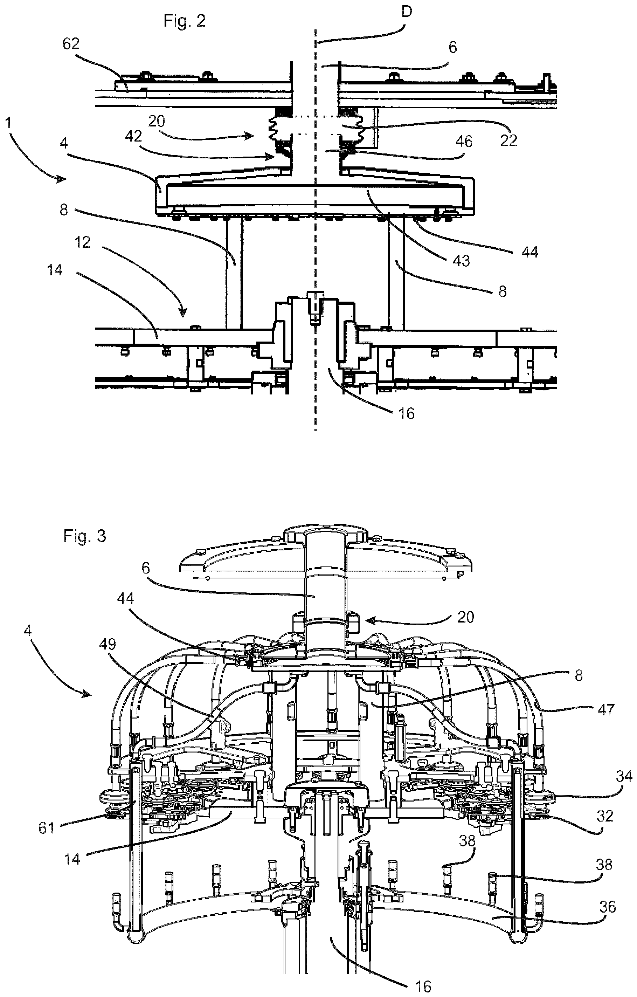

Fig. 2 zeigt eine schematische Darstellung einer erfindungsgemäßen Vorrichtung. Auch hier ist wieder die Zuführleitung 6 vorgesehen, welche stationär an einem Deckel 62 der Vorrichtung angeordnet ist. Das Bezugszeichen 42 kennzeichnet wiederum den Eingang, der hier mit der Verteilereinrichtung 4 dreht. Das Bezugszeichen 44 kennzeichnet einen Ausgang. Das fließfähige Medium wird ausgehend von dem Eingang 42 über einen Verteilerraum auf diese Vielzahl von Ausgängen 44 verteilt. Das Bezugszeichen 43 kennzeichnet einen Sammelraum bzw. eine Sammelkammer, welche das von dem Eingang 42 kommende fließfähige Medium aufnimmt und auf die einzelnen Ausgänge 44 verteilt.

Das Bezugszeichen 12 kennzeichnet wieder die Transporteinrichtung, und das Bezugszeichen 14 den Träger, an dem die Behältnisse gehalten werden. Das Bezugszeichen 16 kennzeichnet eine Antriebswelle, mittels der der Träger 14 in Drehung versetzt werden kann.

Die Verteilereinrichtung 4 ist hier mittels Stützstangen 8 direkt und auch drehfest an dem Träger 14 angeordnet. Auf diese Weise wird die Verteilereinrichtung auch durch diesen Träger 14 gestützt. Die H2O2 Verteilerkammer 4 wird auf diese Weise fest mit dem rotierenden Behandlungsrad bzw. Transportrad verbunden. Dadurch ist keine Lagerung mehr notwendig.

Die Verbindung zwischen stehenden Dach und drehender Verteilerkammer erfolgt über einen Balg 22 mit Wellendichtring. Der Balg 22 übernimmt den Toleranzausgleich und der Wellendichtring die Abdichtung. Über eine Drehmomentstütze wird ein Mitdrehen des Wellendichtrings vermieden.

Entsprechend kennzeichnet das Bezugszeichen 20 eine Verbindungsstelle, um den Eingang 42 drehbar gegenüber der Zuführleitung 6 zu "lagern". Genauer bezieht sich hier das Bezugszeichen 22 auf einen Faltenbalg, der ein Bestandteil dieser Abdichteinrichtung ist. Das Bezugszeichen D kennzeichnet eine Drehachse, bezüglich derer die Verteilereinrichtung 4 und auch der Träger 14 drehbar sind. Anstelle eines Faltenbalgs 22 mit Wellendichtring/Gleitlagerbuchse kann jedoch auch eine Lippendichtung Anwendung finden.

Fig. 3 zeigt eine detailliertere Darstellung einer erfindungsgemäßen Vorrichtung. Auch hier ist wieder die stehende Zuführleitung 6 vorgesehen und die Lagereinrichtung 20. Das Bezugszeichen 47 kennzeichnet auch hier wieder Zuführleitungen, welche das Sterilisationsmedium zu den (nicht gezeigten) Behältnissen führen. Die Behältnisse sind hier bevorzugt mittels Haltereinrichtungen 32 an den Träger 14 befestigt. Die Bezugszeichen 38 kennzeichnen Düsen, über welche ein Sterilisationsmittel austreten und die Außenseite der Preformen sterilisieren kann. Das Bezugszeichen 36 kennzeichnet einen Ringkanal, der mit dem Sterilisationsmittel beaufschlagt werden kann und/oder der das Sterilisationsmittel zuleitet und/führt.

Das Bezugszeichen 34 kennzeichnet weitere Beaufschlagungseinrichtungen, welche zumindest die Innenseite der Behältnisse mit einem Sterilisationsmittel beaufschlagen. Dabei kann vorteilhaft jeder Haltereinrichtung für ein Behältnis eine solche Beaufschlagungseinrichtung 34 zugeordnet sein. Die Bezugszeichen 49 kennzeichnen weitere Zuführleitungen, welche das fließfähige Medium zu weiteren Beaufschlagungseinrichtungen 36 fördern. Die Beaufschlagungseinrichtungen 36 dienen der Außensterilisation der Vorformlinge und werden aus der gleichen H2O2-Verteilerkammer gespeist. Damit sind bevorzugt wenigstens zwei Zuführleitungen vorgesehen, welche das fließfähige Medium zu unterschiedlichen Bereichen von Behältnissen führen. Das Bezugszeichen 61 kennzeichnet weitere Leitungen, welche hier das fließfähige Medium in vertikaler Richtung fördern. Die Verteilereinrichtung 4 kann dabei Schaltmittel (nicht gezeigt) aufweisen, welche eine wahlweise Versorgung der Zuführleitungen 49 bzw. der Zuführleitungen 47 mit dem fließfähigen Medium ermöglichen. Bevorzugt sind jedoch diese Zuführleitungen 49 und 47 gleichzeitig im Einsatz und werden bevorzugt auch aus derselben Verteilkammer gespeist.

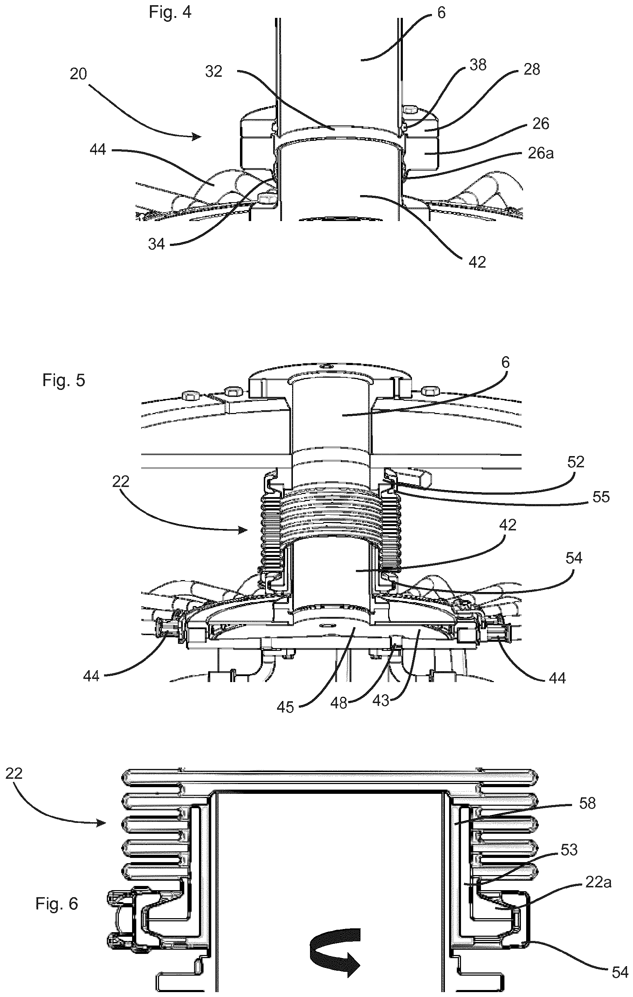

Wie in Fig. 4 gezeigt, wird bei der in Fig. 3 gezeigten Ausgestaltung als Abdichteinrichtung 20 eine Lippendichtung verwendet. Diese Lippendichtung weist dabei einen Befestigungsring 28 auf, an dem das eigentliche Dichtmittel 26 befestigt ist. Diese Lippendichtung 26 weist dabei eine Dichtlippe auf, welche bevorzugt auch eine Verschiebung der Bauteile zueinander (welche beispielsweise durch Wärmedehnung oder Rundlauffehler auftreten kann) zulässt. Diese Dichtlippe ist mit dem Bezugszeichen 26a gekennzeichnet. Die Hauptfunktion der Abdichteinrichtung besteht darin, die Weiterleitung des fließfähigen Mediums abzudichten. Daneben ist es jedoch auch denkbar, dass diese Abdichteinrichtung auch eine gewisse Schnittstellenfunktion aufnimmt, um die Drehung des Eingangs 42 gegenüber der Zuführeinrichtung zu lagern. Allerdings ist die Abdichteinrichtung bevorzugt nicht in der Lage, ausreichende Kräfte entlang der Drehachse D aufzunehmen, um die Verteilereinrichtung 4 alleine zu halten.

Die Fig. 5 und 6 zeigen eine weitere Ausgestaltung einer erfindungsgemäßen Vorrichtung, wobei hier die Abdichteinrichtung als Faltenbalg mit Gleitlagerbuchse ausgeführt ist. Der Faltenbalg 22 ist hier dazu geeignet, eine Relativbewegung der Verteilereinrichtung bzw. der Verteilerscheibe 45 gegenüber dem oberen Flansch 52 zu kompensieren. Derartige Relativbewegungen können beispielsweise durch eine Wärmedehnung der Einhausung auftreten. Das Bezugszeichen 54 bezieht sich auf eine Klammer, welche ein unteres Ende 22a des Faltenbalgs 22 an einer Halterung 53 befestigt. Das Bezugszeichen 58 kennzeichnet eine Gleitlagerbuchse, welche hier stehend ausgebildet ist. Damit fungiert das gesamte Gleitlager als Spaltdichtung und führt den Faltenbalg 22 in seinem unteren Bereich. Die Klammern 54 und 55 (Fig. 5) dienen hier bevorzugt auch als Schnellverschluss. Auf diese Weise lässt sich die gesamte Gleitlagerbuchse in kurzer Zeit auswechseln. Auch hat sich gezeigt, dass ein Leckageverlust nicht größer ist als bei Vorrichtungen nach dem internen Stand der Technik der Anmelderin. Auch in der oben erwähnten Verteilerscheibe 45 können Öffnungen 48 zum Abführen des fließfähigen Mediums vorgesehen sein. Ausgehend von diesen Öffnungen gelangt das fließfähige Medium zu den Zuführleitungen 49 und damit zu dem Ringkanal 36.

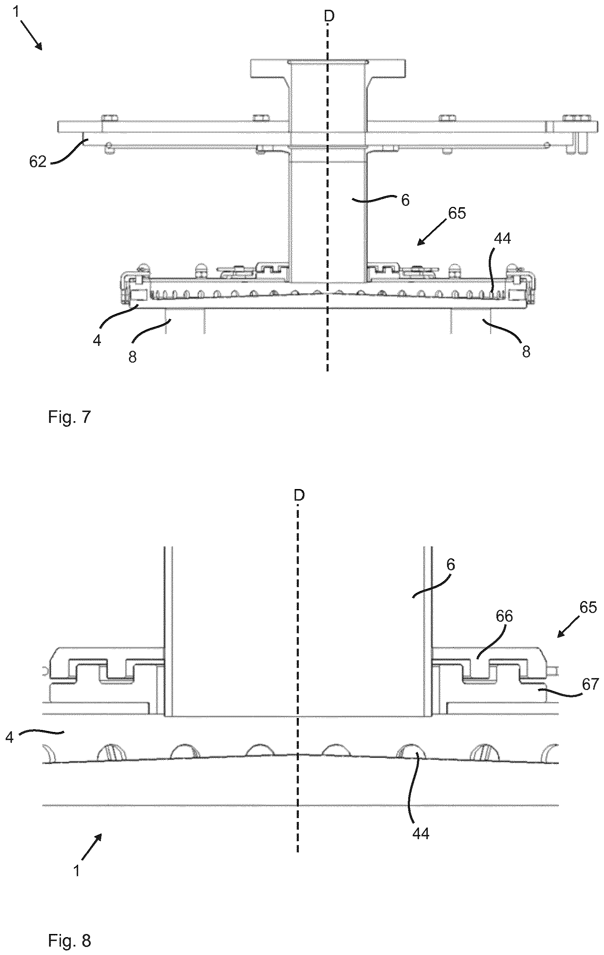

Die Figuren 7 und 8 zeigen eine weitere Ausführungsform einer erfindungsgemäßen Vorrichtung, wobei hier als Abdichtung zwischen der Zuführleitung 6 und der Verteilereinrichtung 4 eine Labyrinth-Dichtung 65 vorgesehen ist. Wie insbesondere aus der Figur 8 erkennbar ist, ist die Labyrinth-Dichtung 65 bevorzugt zweiteilig ausgebildet, wobei ein erster Teil 66 der Labyrinth-Dichtung an der stehenden Zuführleitung 6 angeordnet ist und ein zweiter Teil 67 der Labyrinth-Dichtung an der drehenden Verteilereinrichtung 4. Das Bezugszeichen 8 bezieht sich dabei wiederum auf Stützstangen, so dass auch in dieser Ausführungsform die Verteilereinrichtung 4 an dem Träger angeordnet ist. Auf diese Weise ist auch hier die Verteilerkammer 4 mit dem rotierenden Behandlungsrad bzw. Transportrad verbunden, wodurch die Notwendigkeit einer Lagerung entfällt.

### Bezugszeichenliste

- 1: Vorrichtung
- 2: Gehäuse
- 4: Verteilereinrichtung
- 6: Zuführleitung
- 8: Stützstangen
- 12: Transporteinrichtung mit drehbarem Träger
- 14: Träger
- 16: Antriebswelle
- 20: Lagerungseinrichtung/Abdichteinrichtung
- 22: Faltenbalg
- 22a: unteres Ende des Faltenbalgs
- 26: Dichtmittel
- 26a: Dichtlippe
- 28: Befestigungsring
- 32: Halteeinrichtungen
- 34: Weitere Beaufschlagungseinrichtungen
- 36: Ringkanal
- 38: Düsen
- 42: Eingang
- 44: Ausgänge
- 45: Verteilerscheibe
- 47: Zuführleitungen
- 48: Öffnungen
- 49: Zuführleitungen
- 52: Oberer Flansch
- 53: Halterung
- 54: Klammer 1
- 55: Klammer 2
- 58: Gleitlagerbuchse
- 61: Leitungen
- 65: Labyrinth-Dichtung
- 66: erster Teil der Labyrinth-Dichtung 65
- 67: zweiter Teil der Labyrinth-Dichtung 65
- 120: Wälzlager, Lagerungseinrichtung
- 122: Lagerkörper
- D: Drehachse

## Patentansprüche

1. Vorrichtung (1) zum Verteilen fließfähiger Medien auf Behältnisse mit einem Gehäuse (2), mit einer wenigstens teilweise innerhalb des Gehäuses (2) angeordneten Transporteinrichtung (12) zum Transportieren der Behältnisse, wobei diese Transporteinrichtung einen drehbaren Träger (14) aufweist, mit einer innerhalb dieses Gehäuses (2) angeordneten Verteilereinrichtung (4), welche wenigstens einen Eingang (42) für das fließfähige Medium und eine Vielzahl von Ausgängen (44), auf welche das fließfähige Medium verteilbar ist, aufnimmt und mit einer Zuführleitung (6), welche dazu geeignet und bestimmt ist, dem Eingang (42) das fließfähige Medium zuzuführen,
wobei die Verteilereinrichtung (4) drehfest mit dem Träger (14) verbunden ist, wobei sich die Zuführeinrichtung (6) durch einen Deckenabschnitt der Vorrichtung (1) erstreckt und die Zuführleitung (6) gegenüber der Deckenwandung stationär ist und zwischen der Zuführleitung (6) und der Verteilereinrichtung (4) eine Abdichteinrichtung (20) angeordnet ist, **dadurch gekennzeichnet, dass** die Abdichteinrichtung (20) auch dazu geeignet und dazu bestimmt ist, dass zu verteilende fließfähige Medium von der Zuführleitung (6) zu dem Eingang zu leiten.

2. Vorrichtung (1) nach Anspruch 1,
**dadurch gekennzeichnet, dass**
die Zuführleitung (6) wenigstens abschnittsweise drehfest angeordnet ist und die Verteilereinrichtung (4) gegenüber der Zuführleitung (6) drehbar ist.

3. Vorrichtung (1) nach Anspruch 1,
**dadurch gekennzeichnet, dass**
die Transporteinrichtung Bestandteil einer Behandlungseinrichtung, insbesondere einer Einrichtung zum Sterilisieren von Preforms, ist.

4. Vorrichtung (1) nach Anspruch 1,
**dadurch gekennzeichnet, dass**
das fließfähige Medium gasförmiges H2O2 ist.

5. Vorrichtung (1) nach Anspruch 1,
**dadurch gekennzeichnet, dass**
zumindest die Vorrichtung in ihrer Gesamtheit in einem Reinraum angeordnet ist.

6. Vorrichtung (1) nach wenigstens einem der vorangegangenen Ansprüche,
**dadurch gekennzeichnet, dass**
der Eingang (42) eine drehfest an der Verteilereinrichtung (4) angeordnete Eingangsleitung (46) aufweist, welche in Strömungsverbindung mit der Zuführleitung (6) bringbar ist.

7. Vorrichtung (1) nach Anspruch 6,
**dadurch gekennzeichnet, dass**
zwischen der Eingangsleitung (46) und der Zuführleitung eine Abdichteinrichtung (20) angeordnet ist.

8. Vorrichtung (1) nach wenigstens einem der Ansprüche,
**dadurch gekennzeichnet, dass**
die Abdichteinrichtung (20) ein entlang der Drehachse (D) elastisches Element (22) und bevorzugt einen Faltenbalg aufweist.

9. Vorrichtung (1) nach Anspruch 8,
**dadurch gekennzeichnet, dass**
das elastische Element die Zuführleitung (6) mit dem Eingang (42) der Verteilereinrichtung (4) und insbesondere mit einer Eingangsleitung (46) des Eingangs verbindet.

10. Vorrichtung nach wenigstens einem der vorangegangenen Ansprüche,
**dadurch gekennzeichnet, dass**
die Abdichteinrichtung (20) eine Lagerungseinrichtung zum drehbaren Lagern wenigstens eines Elements des Eingangs (42) oder der Zuführleitung aufweist.

11. Vorrichtung nach Anspruch 7,
**dadurch gekennzeichnet, dass**
die Lagerungseinrichtung (20) und/oder die Abdichteinrichtung (20) als Wellendichtring ausgeführt ist.

12. Vorrichtung nach Anspruch 1,
**dadurch gekennzeichnet, dass**
die Zuführleitung (6) und die Verteilereinrichtung (4) berührungslos ausgebildet sind.

13. Vorrichtung nach Anspruch 12,
**dadurch gekennzeichnet, dass**
eine Abdichtung zwischen der Zuführleitung (6) und der Verteilereinrichtung (4) durch eine berührungslose Labyrinth-Dichtung (65) erfolgt.

14. Verfahren zum Behandeln und insbesondere zum Sterilisieren von Behältnissen mit einer Vorrichtung (1) gemäß einem der Ansprüche 1 bis 13 zum Verteilen fließfähiger Medien, wobei die Behältnisse mittels der Transporteinrichtung (12) transportiert und während dieses Transports mit einem fließfähigen Medium beaufschlagt werden und wobei die Transporteinrichtung (12) den drehbaren Träger (14) aufweist, mittels dessen die Behältnisse entlang einer im Wesentlichen kreisförmigen Bahn transportiert werden und dieser Träger (14) sich innerhalb des Gehäuses (2) befindet, wobei mittels der Zuführleitung (6) der ebenfalls innerhalb dieses Gehäuses (2) befindlichen Verteilereinrichtung (4) das fließfähige Medium über den Eingang (42) zugeführt wird und die Verteilereinrichtung (4) das fließfähige Medium auf die Ausgänge (44) verteilt, wobei die Verteilereinrichtung (4) drehfest mit dem drehbaren Träger (14) verbunden ist, wobei sich die Zuführeinrichtung (6) durch den Deckenabschnitt der Vorrichtung (1) erstreckt und die Zuführleitung (6) gegenüber der Deckenwandung stationär ist und zwischen der Zuführleitung (6) und der Verteilereinrichtung (4) die Abdichteinrichtung (20) angeordnet ist, welche auch dazu geeignet und dazu bestimmt ist, dass zu verteilende fließfähige Medium von der Zuführleitung (6) zu dem Eingang zu leiten.

## Claims

1. Apparatus (1) for distributing flowable media to containers with a housing (2), with a transport device (12) for transporting the containers which is arranged at least partially inside the housing (2), wherein this transport device has a rotatable carrier (14) with a distributor device (4) which is arranged inside this housing (2) which accommodates at least one inlet (42) for the flowable medium and a plurality of outlets (44) to which the flowable medium can be distributed, and with a supply line (6) which is suitable and intended for supplying the flowable medium to the inlet (42), wherein the distributor device (4) is connected non-rotatably to the carrier (14), wherein supply device (6) extends through a cover section of the apparatus (1) and the supply line (6) is stationary with respect to the cover wall and a sealing device (20) is arranged between the supply line (6) and the distributor device (4),
**characterised in that**
the sealing device (20) is suitable and intended for guiding the flowable medium to be distributed from the supply line (6) to the inlet.

2. Apparatus (1) according to claim 1,
**characterised in that**
the supply line (6) is arranged, at least partially, non-rotatably and the distributor device (4) is rotatable relative to the supply line (6).

3. Apparatus (1) according to claim 1,
**characterised in that**
the transport device is a component of a processing device, in particular a device for sterilising preforms.

4. Apparatus (1) according to claim 1,
**characterised in that**
the flowable medium is gaseous H₂O₂.

5. Apparatus (1) according to claim 1,
**characterised in that**
at least the apparatus in its entirety is arranged in a clean room.

6. Apparatus (1) according to at least one of the preceding claims,
**characterised in that**
the inlet (42) has an inlet line (46) which is arranged non-rotatably on the distributor device (4) and can be fluidically connected to the supply line (6).

7. Apparatus (1) according to claim 6,
**characterised in that**
a sealing device (20) is arranged between the inlet line (46) and the supply line.

8. Apparatus (1) according to at least one of the claims, **characterised in that**
the sealing device (20) has an elastic element (22) along the axis of rotation (D) and preferably a bellows.

9. Apparatus (1) according to claim 8,
**characterised in that**
the elastic element connects the supply line (6) to the inlet (42) of the distributor device (4) and in particular to an inlet line (46) of the inlet.

10. Apparatus according to at least one of the preceding claims, **characterised in that**
the sealing device (20) has a bearing device for rotatable support of at least one element of the inlet (42) or of the supply line.

11. Apparatus according to claim 7,
**characterised in that**
the bearing device (20) and/or the sealing device (20) is configured as a shaft sealing ring.

12. Apparatus according to claim 1,
**characterised in that**
the supply line (6) and the distributor device (4) are constructed without contact.

13. Machine according to claim 12,
**characterised in that**
a sealing between the supply line (6) and the distributor device (4) takes place by a contactless labyrinth seal (65).

14. Method for processing and in particular sterilizing containers having an apparatus (1) according to at least one of the preceding claims 1 to 13 for distributing flowable media, wherein the containers are transported by the transport device (12) and being acted upon during this transport by a flowable medium, and wherein the transport device (12) has the rotatable carrier (14), by which the containers are transported along a substantially circular path and this carrier (14) is located inside the housing (2), wherein the flowable medium is fed by the supply line (6) to the distributor device (4) likewise located inside this housing (2) via an inlet (42) and the distributor device (4) distributes the flowable medium to the outlets (44), wherein the distributor device (4) is non-rotatably connected to the rotatable carrier (14), wherein the supply device (6) extends through the cover section of the apparatus (1) and the supply line (6) is stationary with respect to the cover wall and the sealing device (20) is arranged between the supply line (6) and the distributor device (4), which is also suitable and intended for guiding the flowable medium to be distributed from the supply line (6) to the inlet.

## Revendications

1. Dispositif (1) de répartition de milieux fluides sur des récipients avec un boîtier (2), avec un système de transport (12) disposé au moins en partie à l'intérieur du boîtier (2) destiné à transporter les récipients, dans lequel ledit système de transport présente un support (14) rotatif, avec un système de répartition (4) disposé à l'intérieur dudit boîtier (2), lequel accueille au moins une entrée (42) pour le milieu fluide et une pluralité de sorties (44), sur lesquelles le milieu fluide peut être réparti, et avec une conduite d'amenée (6), laquelle est adaptée pour et se destine à amener le milieu fluide à l'entrée (42),
dans lequel le système de répartition (4) est relié de manière solidaire en rotation au support (14), dans lequel le système d'amenée (6) s'étend à travers une section de couvercle du dispositif (1) et la conduite d'amenée (6) est stationnaire par rapport à la paroi de couvercle et un système d'étanchéification (20) est disposé entre la conduite d'amenée (6) et le système de répartition (4),
**caractérisé en ce que**
le système d'étanchéification (20) est également adapté pour et se destine à acheminer du milieu fluide à répartir depuis la conduite d'amenée (6) vers l'entrée.

2. Dispositif (1) selon la revendication 1,
**caractérisé en ce que**
la conduite d'amenée (6) est disposée de manière solidaire en rotation au moins par endroits et le système de répartition (4) peut être tourné par rapport à la conduite d'amenée (6).

3. Dispositif (1) selon la revendication 1,
**caractérisé en ce que**
le système de transport fait partie intégrante d'un système de traitement, en particulier d'un système de stérilisation de préformes.

4. Dispositif (1) selon la revendication 1,
**caractérisé en ce que**
le milieu fluide est du H2O2 gazeux.

5. Dispositif (1) selon la revendication 1,
**caractérisé en ce que**
au moins le dispositif est disposé dans sa totalité dans une salle blanche.

6. Dispositif (1) selon au moins l'une quelconque des revendications précédentes,
**caractérisé en ce que**
l'entrée (42) présente une conduite d'entrée (46) disposée de manière solidaire en rotation sur le système de répartition (4), laquelle peut être amenée en communication fluidique avec la conduite d'amenée (6).

7. Dispositif (1) selon la revendication 6,
**caractérisé en ce que**
un système d'étanchéification (20) est disposé entre la conduite d'entrée (46) et la conduite d'amenée.

8. Dispositif (1) selon au moins l'une quelconque des revendications précédentes,
**caractérisé en ce que**
un système d'étanchéification (20) présente un élément élastique (22) le long de l'axe de rotation (D) et de manière préférée un soufflet.

9. Dispositif (1) selon la revendication 8,
**caractérisé en ce que**
l'élément élastique relie la conduite d'amenée (6) à l'entrée (42) du système de répartition (4) et en particulier à une conduite d'entrée (46) de l'entrée.

10. Dispositif selon au moins l'une quelconque des revendications précédentes,
**caractérisé en ce que**
le système d'étanchéification (20) présente un système de montage destiné à monter de manière rotative au moins un élément de l'entrée (42) ou de la conduite d'amenée.

11. Dispositif selon la revendication 7,
**caractérisé en ce que**
le système de montage (20) et/ou le système d'étanchéification (20) sont réalisés en tant que bagues d'étanchéité d'arbre.

12. Dispositif selon la revendication 1,
**caractérisé en ce que**
la conduite d'amenée (6) et le système de répartition (4) sont réalisés sans contact.

13. Dispositif selon la revendication 12,
**caractérisé en ce que**
une étanchéification entre la conduite d'amenée (6) et le système de répartition (4) est effectuée par un joint d'étanchéité à labyrinthe (65) sans contact.

14. Procédé de traitement et en particulier de stérilisation de récipients avec un dispositif (1) selon l'une quelconque des revendications 1 à 13 de répartition de milieux fluides, dans lequel les récipients sont transportés au moyen du système de transport (12) et sont soumis à l'action d'un milieu fluide pendant ledit transport et dans lequel le système de transport (12) présente le support (14) rotatif, au moyen duquel les récipients sont transportés le long d'une trajectoire sensiblement circulaire et ledit support (14) se trouve à l'intérieur du boîtier (2), dans lequel le milieu fluide est amené par l'intermédiaire de l'entrée (42) au système de répartition (4) se trouvant également à l'intérieur du boîtier (2) au moyen de la conduite d'amenée (6) et le système de répartition (4) répartit le milieu fluide sur les sorties (44),
dans lequel le système de répartition (4) est relié de manière solidaire en rotation au support (14) rotatif, dans lequel le système d'amenée (6) s'étend à travers la section de couvercle du dispositif (1) et la conduite d'amenée (6) est stationnaire par rapport à la paroi de couvercle et le système d'étanchéification (20), qui est adapté pour et se destine à acheminer du milieu fluide à répartir depuis la conduite d'amenée (6) vers l'entrée, est disposé entre la conduite d'amenée (6) et le système de répartition (4).
